# EUROPEAN PATENT SPECIFICATION

(11) **EP 2 097 468 B1**
(45) Date of publication and mention of the grant of the patent: **25.08.2010**
(21) Application number: 07847101.8
(22) Date of filing: 02.11.2007
(51) Int. Cl.: C08G 61/08, A61K 8/00

(54) **COMPOSITION COMPRISING BRUSH COPOLYMER FOR TREATING HAIR**
ZUSAMMENSETZUNG MIT BÜRSTEN-COPOLYMER ZUR HAARBEHANDLUNG
COMPOSITION COMPRENANT UN COPOLYMÈRE EN BROSSE DESTINÉE À TRAITER LES CHEVEUX

(30) Priority: 27.11.2006 EP 06124834
(43) Date of publication of application: 09.09.2009
(73) Proprietor: Unilever PLC, London EC4Y 0DY (GB); Unilever N.V., 3013 AL Rotterdam (NL)
(72) Inventor: BURRY, Jason Shaun, Bebington, Wirral Merseyside CH63 3JW (GB); CHENG, Chong, Saint Louis, mo 63112 (US); EVANS, Richard Livesey, Bebington, Wirral Merseyside CH63 3JW (GB); KHOSHDEL, Ezat, Bebington, Wirral Merseyside CH63 3JW (GB); WOOLEY, Karen Lynn, Chesterfield, MO 63005 (US)
(74) Representative: Tansley, Sally Elizabeth
(86) International application number: PCT/EP2007/061816
(87) International publication number: WO 2008/064973

(56) References cited:
- WO-A-03/040218
- WO-A-2006/024184
- MAHANTHAPPA M K ET AL: "SYNTHESIS OF ABA TRIBLOCK COPOLYMERS BY A TANDEM ROMP-RAFT STRATEGY" 20 September 2005 (2005-09-20), MACROMOLECULES, ACS, WASHINGTON, DC, US, PAGE(S) 7890-7894 , XP001235071 ISSN: 0024-9297 the whole document
- PATTON DEREK L ET AL: "A versatile synthetic route to macromonomers via RAFT polymerization" MACROMOLECULES; MACROMOLECULES DEC 12 2006, vol. 39, no. 25, 17 November 2006 (2006-11-17), pages 8674-8683, XP002429067
- SCHIMETTA M ET AL: "RING-OPENING METATHESIS POLYMERIZATION FO THE BIS(METHYL CARBONATE)AND BIS(S-METHYL DITHIOCARBONATE) OF NORBORNENE AND THERMAL CONVERSION TO POLY(CYCLOPENTADIENYLENEVINYLENE)" MACROMOLECULES, ACS, WASHINGTON, DC, US, vol. 27, no. 14, 4 July 1994 (1994-07-04), pages 3769-3772, XP000457206 ISSN: 0024-9297

## Description

### Field of the Invention

This invention relates to compositions, copolymeric materials and methods for delivering benefit agents from these compositions.

### Background

Personal care compositions contain a range of agents that enhance the body or hair. Benefit agents, such as perfumes, silicones, waxes, flavours, vitamins and fibre active agents are expensive and generally less effective when employed at high levels in personal care compositions, cleaning compositions. As a result, there is a desire to maximise, the effectiveness of such benefit agents. One method of achieving such object is to improve the delivery efficiencies of such benefit agents. Unfortunately, it is difficult to improve the delivery efficiencies of benefit agents as such agents may be lost due to the agents' physical or chemical characteristics, or such agents may be incompatible with other compositional components or the sites that are treated.

Mahanthappa MK et al. disclose in Macromolecules, 2005, 38, 7890-7894 the synthesis of ABA-type triblock copolymers of mechanistically incompatible monomer pairs such as 1,5-cyclooctadiene with styrene and acrylate monomers by a tandem ring-opening metathesis polymerisation (ROMP) and reversible addition fragmentation transfer polymersation (RAFT) method.

Patton DL et al. disclose in Macromolecules 2006, 39, 8674-8683 the preparation of functional macromonomers using reversible addition-fragmentation chain transfer (RAFT) polymerisation. The macromonomer utility was demonstrated by synthesing a poly(norbornene-*g*-poly methyl methacrylate (PMMA) copolymer based on the ring-opening metathesis polymerisation (ROMP) of the norbornenyl-functionalised PMMA macromonomer.

WO 2006/024184 A discloses a process for the production of controlled-release aqueous compositions comprising an active ingredient. The polymer particles are obtainable by ring-opening metathesis polymerisation (ROMP) of a monomer.

Accordingly, there is a need for a benefit agent containing delivery particles that provide improved benefit agent delivery efficiency.

The present invention relates to a benefit agent delivery system. When employed in compositions, for example cleaning (e.g. shampoo or body wash) or conditioning compositions or leave-on products for skin such as the scalp and other body sites, the benefit agent delivery system increases the efficiency of benefit agent delivery, thereby allowing reduced amounts of benefit agents to be employed. In addition by allowing the amount of benefit agent to be reduced, the benefit agent delivery system allows a broad range of benefit agents to be employed

### Description of the invention

The present invention relates to compositions comprising a brush copolymer of formula 1: 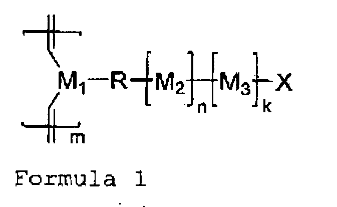 in which M₁ is a unit obtainable from ring opening metathesis polymerisation (ROMP) ;
R is an alkyl, ether, ester or aryl unit;
M₂ and M₃ are independently selected from units obtainable by reversible addition fragmentation chain transfer polymerization(RAFT);
X is a terminal unit selected from the group consisting of dithioester, trithiocarbonate, xanthate; and m is an integer from 2 to 1 million, n is an integer from 2 to 500,000 and k is an integer from 2 to 500,000; in which incorporated within the core shell brush copolymer is a benefit agent selected from hair styling polymers, colourants, hair conditioners, hair cleansers, hair growth promoters, permanent wave compounds, hair relaxers, amino acids, vitamins, hair straighteners, hair growth stimulants, antibacterial compounds, antifungal compounds, anti-inflammatory compounds, sunscreens and mixtures thereof.

The use of the above mentioned copolymers to deliver a benefit agent to the hair is also described as is a method of treating the hair by application to the hair a composition the above mentioned copolymers

### Detailed Description

The present invention relates as core-shell brush copolymers for delivering benefit agents. Preferably the brush copolymers are in the form of a nanocage structure.

### Core-shell brush copolymers

The present invention relates to a composition comprising a brush copolymer of formula 1: 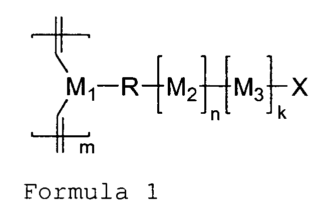 in which M₁ is a unit obtainable from ring opening metathesis polymerisation (ROMP) preferably a unit obtainable from the ring opening metathesis polymerisation (ROMP) of cyclooctadiene or norbornene.

R is an alkyl, ether, ester or aryl unit;
M₂ and M₃ are independently selected from units obtainable by reversible addition fragmentation chain transfer polymerization(RAFT), preferably units obtainable by reversible addition fragmentation chain transfer polymerization(RAFT) of styrene, isoprene, methyl acrylate, *tert*-butyl acrylate, dimethyl acryamide, acrylic acid, acrylonitrile, methyl methacrylate, maleic anhydride, vinyl acetate, vinyl pyridine orvinyl phenyl ketone;
X is a terminal unit selected from the group consisting of dithioester, trithiocarbonate, xanthate; and m is an integer from 2 to 1 million, preferably from 10 to 500,000, more preferably from 100 to 200,000" n is an integer from 2 to 500,000, preferably from 10 to 250,000, more preferably from 20 to 100,000 and k is an integer from 2 to 500,000, preferably from 10 to 250,000, more preferably from 20 to 100,000

Preferably the core shell brush copolymer has the structure of formula 2: 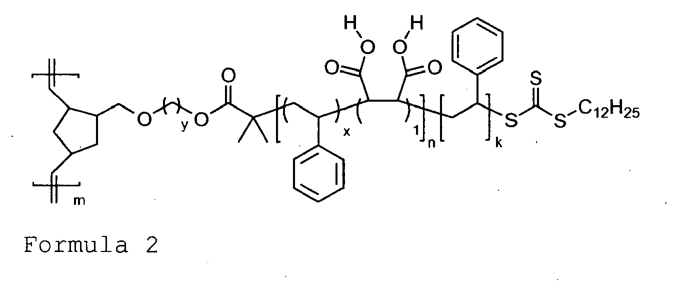 in which m is an integer from 2 to 1 million, and y is an integer from 2 to 18, x is an integer from 1 to 100, n is an integer from 2 to 500,000 and k is an integer from 2 to 500,000.

The invention further relates to a core-shell brush copolymer of formula 3 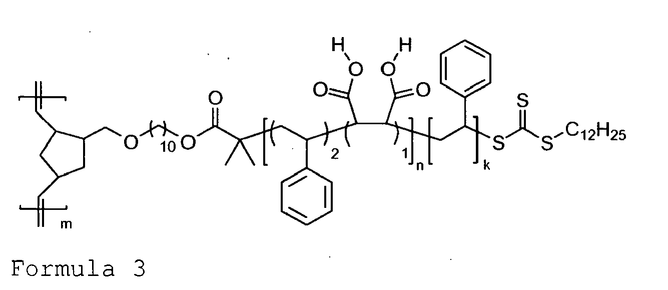 in which m is an integer from 2 to 1 million, n is an integer from 2 to 500,000 and k is an integer from 2 to 500,000.

A further aspect of the invention is a core-shell brush copolymer of formula 4 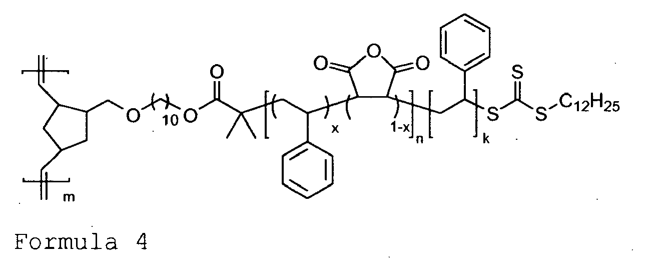 in which m is an integer from 2 to 1 million, x is a value from 0 to 1, n is an integer from 2 to 500,000 and k is an integer from 2 to 500,000.

The core-shell brush copolymers of the invention are preferably particulate in nature, having dimensions ranging from 2 to 2000 nm and aspect ratios from 1 to 10000, as measured by dynamic light scattering, atomic force microscopy, transmission electron microscopy, or other standard means that are known to those in the art.

The core-shell brush copolymer is manufactured using the step of tandem ROMP (ring opening metathesis polymerisation) and RAFT (reversible addition fragmentation chain transfer polymerization).

The preferred reaction scheme is as follows:

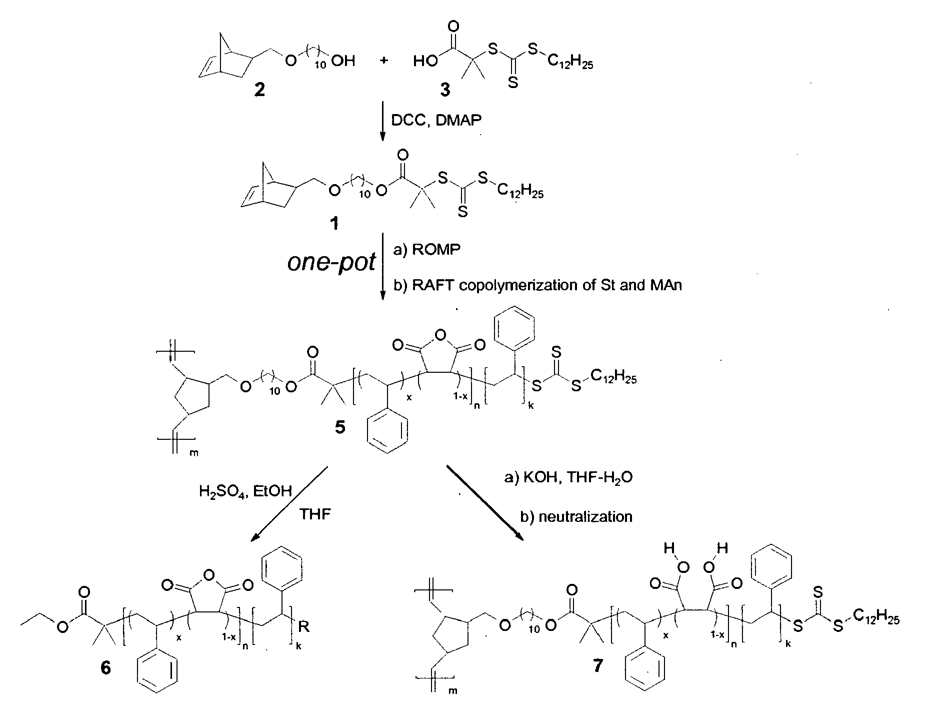

The core shell brush copolymer may be further polymerised so that it forms a nanocage. The nanocage is prepared by internal cross linking, in particulate internal cross linking. In the context of the present invention a nanocage can be defined as copolymeric polymer lined in such a manner that it forms a shell like structure. A highly preferable use of this shell like structure is for encapsulating material.

Incorporated within the core-shell brush copolymer is a benefit agent.

The core shell brush copolymer is preferably present (with the benefit agent) the total composition in an amount of from 0.01 to 50 wt%, more preferably from 0.2 to 20 wt%, most preferably from 0.5 to 10 wt%.

### Benefit agents:

The benefit agents are selected from hair styling polymers, colourants, hair conditioners, hair cleansers, hair growth promoters, permanent wave compounds, hair relaxers, amino acids, vitamins, hair straighteners, hair growth stimulants, hair growth retardants, hair shedding inhibitors, antibacterial, compounds, antifungal compounds, anti-inflammatory compounds, sunscreens and mixtures thereof.

Especially preferred is an antifungal compound, an antibacterial compound, an anti-inflammatory compound, or mixtures thereof. Of particular interest is salicylic acid.

In an alternative aspect of the invention hair growth promoters are of interest as benefit agents. Examples of substances which themselves possess the ability to stimulate or increase hair growth include benzalkonium chloride, benzethonium chloride, phenol estradiol, diphenhydramine hydrochloride, chlorpheniramine maleate, chlorophyllin derivatives, cholesterol, salicylic acid, cystine, red pepper tincture, benzyl nicotinate, dl-menthol, peppermint oil, calcium pantothenate, panthenol, castor oil, hinokitiol, prednisolone, minoxidil, saw palmetto extract, nettle root extract, capsaicin, niacin, ginko biloba, horsetail extract, phospholipid, glycerol oxide esters, cyclodextrin, ketoconazole, ursolic acid, polysorbate, 1,4,3,6-dianhydro-2,5-d-o-methyl-d-glucitol, milk thistle, methyl nicotinate, finasteride, azelaic acid, medroxyprogestorone, dutasteride, topical Hedgehog agonists; adenosine, trans-3,4'-dimethyl-3-hydorxyflavanone and resorcinol.

These benefit agents are incorporated into the composition preferably in an amount of 0.0001 to 5 % based on the total weight of the composition.

### Product Form

Compositions of the invention are preferably personal care compositions, more preferably they are hair treatment compositions.

Compositions of the invention can be rinsed off or left on the hair, typically they are "rinse-off" compositions to be applied to the hair and then rinsed away.

A particularly preferred product form is a shampoo composition.

### Shampoo Composition

Shampoo compositions of the invention are generally aqueous, i.e. they have water or an aqueous solution or a lyotropic liquid crystalline phase as their major component. Suitably, the composition will comprise from 50 to 98%, preferably from 60 to 90% water by weight based on the total weight of the composition.

### Anionic Cleansing Surfactant

Shampoo compositions as claimed in the invention will generally comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, alkyl ether sulphosuccinates, N-alkyl sarcosinates, alkyl phosphates, alkyl ether phosphates, and alkyl ether carboxylic acids and salts thereof, especially their sodium, magnesium, ammonium and mono-, di- and triethanolamine salts. The alkyl and acyl groups generally contain from 8 to 18, preferably from 10 to 16 carbon atoms and may be unsaturated. The alkyl ether sulphates, alkyl ether sulphosuccinates, alkyl ether phosphates and alkyl ether carboxylic acids and salts thereof may contain from 1 to 20 ethylene oxide or propylene oxide units per molecule.

Typical anionic cleansing surfactants for use in shampoo compositions of the invention include sodium oleyl succinate, ammonium lauryl sulphosuccinate, sodium lauryl sulphate, sodium lauryl ether sulphate, sodium lauryl ether sulphosuccinate, ammonium lauryl sulphate, ammonium lauryl ether sulphate, sodium dodecylbenzene sulphonate, triethanolamine dodecylbenzene sulphonate, sodium cocoyl isethionate, sodium lauryl isethionate, lauryl ether carboxylic acid and sodium N-lauryl sarcosinate.

Preferred anionic cleansing surfactants are sodium lauryl sulphate, sodium lauryl ether sulphate(n)EO, (where n is from 1 to 3), sodium lauryl ether sulphosuccinate(n)EO, (where n is from 1 to 3), ammonium lauryl sulphate, ammonium lauryl ether sulphate(n)EO, (where n is from 1 to 3), sodium cocoyl isethionate and lauryl ether carboxylic acid (n) EO (where n is from 10 to 20).

Mixtures of any of the foregoing anionic cleansing surfactants may also be suitable.

The total amount of anionic cleansing surfactant in shampoo compositions of the invention generally ranges from 0.5 to 45%, preferably from 1.5 to 35%, more preferably from 5 to 20% by total weight anionic cleansing surfactant based on the total weight of the composition.

### Further Ingredients

Optionally, a shampoo composition of the invention may contain further ingredients as described below to enhance performance and/or consumer acceptability.

### Co-surfactant

The composition can include co-surfactants, to help impart aesthetic, physical or cleansing properties to the composition.

An example of a co-surfactant is a nonionic surfactant, which can be included in an amount ranging from 0.5 to 8%, preferably from 2 to 5% by weight based on the total weight of the composition.

For example, representative nonionic surfactants that can be included in shampoo compositions of the invention include condensation products of aliphatic (C₈ - C₁₆) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:

RO - (G)ₙ

wherein R is a branched or straight chain alkyl group which may be saturated or unsaturated and G is a saccharide group.

R may represent a mean alkyl chain length of from about C₅ to about C₂₀. Preferably R represents a mean alkyl chain length of from about C₈ to about C₁₂. Most preferably the value of R lies between about 9.5 and about 10.5. G may be selected from C₅ or C₆ monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies from about 1.1 to about 2. Most preferably the value of n lies from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C₁₀-c₁₈ N-alkyl (C₁-C₆) polyhydroxy fatty acid amides, such as the C₁₂-C₁₈ N-methyl glucamides, as described for example in WO 92 06154 and US 5 194 639, and the N-alkoxy polyhydroxy fatty acid amides, such as C₁₀-C₁₈ N-(3-methoxypropyl) glucamide.

A preferred example of a co-surfactant is an amphoteric or zwitterionic surfactant, which can be included in an amount ranging from 0.5 to about 8%, preferably from 1 to 4% by weight based on the total weight of the composition.

Examples of amphoteric or zwitterionic surfactants include alkyl amine oxides, alkyl betaines, alkyl amidopropyl betaines, alkyl sulphobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine, lauryl betaine, cocamidopropyl betaine and sodium cocoamphoacetate.

A particularly preferred amphoteric or zwitterionic surfactant is cocamidopropyl betaine.

Mixtures of any of the foregoing amphoteric or zwitterionic surfactants may also be suitable. Preferred mixtures are those of cocamidopropyl betaine with further amphoteric or zwitterionic surfactants as described above. A preferred further amphoteric or zwitterionic surfactant is sodium cocoamphoacetate.

The total amount of surfactant (including any co-surfactant, and/or any emulsifier) in a shampoo composition of the invention is generally from 1 to 50%, preferably from 2 to 40%, more preferably from 10 to 25% by total weight surfactant based on the total weight of the composition.

### Cationic Polymers

Cationic polymers are preferred ingredients in a shampoo composition of the invention for enhancing conditioning performance.

Suitable cationic polymers may be homopolymers which are cationically substituted or may be formed from two or more types of monomers. The weight average (M_{w}) molecular weight of the polymers will generally be between 100 000 and 2 million daltons, (100 000 and a million atomic mass units (U)). The polymers will have cationic nitrogen containing groups such as quaternary ammonium or protonated amino groups, or a mixture thereof. If the molecular weight of the polymer is too low, then the conditioning effect is poor. If too high, then there may be problems of high extensional viscosity leading to stringiness of the composition when it is poured.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give polymers having a cationic charge density in the required range, which is generally from 0.2 to 3.0 meq/gm. The cationic charge density of the polymer is suitably determined via the Kjeldahl method as described in the US Pharmacopoeia under chemical tests for nitrogen determination.

Suitable cationic polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-C3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic polymers can comprise mixtures of monomer units derived from amine- and/or quaternary ammonium-substituted monomer and/or compatible spacer monomers.

Suitable cationic polymers include, for example:
- cationic diallyl quaternary ammonium-containing polymers including, for example, dimethyldiallylammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and co-polymers of unsaturated carboxylic acids having from 3 to 5 carbon atoms, (as described in U.S. Patent 4,009,256);
- cationic polyacrylamides(as described in WO95/22311).

Other cationic polymers that can be used include cationic polysaccharide polymers, such as cationic cellulose derivatives, cationic starch derivatives, and cationic guar gum derivatives.

Cationic polysaccharide polymers suitable for use in compositions of the invention include monomers of the formula:

A-O-[R-N⁺(R¹) (R²) (R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic counterion.

Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from the Amerchol Corporation, for instance under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimethylammonium chloride (commercially available from Rhodia in their JAGUAR trademark series). Examples of such materials are JAGUAR C13S, JAGUAR C14, JAGUAR C15, JAGUAR C17 and JAGUAR C16 Jaguar CHT and JAGUAR C162.

Mixtures of any of the above cationic polymers may be used.

Cationic polymer will generally be present in a shampoo composition of the invention at levels of from 0.01 to 5%, preferably from 0.05 to 1%, more preferably from 0.08 to 0.5% by total weight of cationic polymer based on the total weight of the composition.

### Suspending Agent

Preferably an aqueous shampoo composition of the invention further comprises a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives, since these impart pearlescence to the composition. Polyacrylic acid is available commercially as Carbopol 420, Carbopol 488 or Carbopol 493. Polymers of acrylic acid cross-linked with a polyfunctional agent may also be used; they are available commercially as Carbopol 910, Carbopol 934, Carbopol 941 and Carbopol 980. An example of a suitable copolymer of a carboxylic acid containing monomer and acrylic acid esters is Carbopol 1342. All Carbopol (trademark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

Mixtures of any of the above suspending agents may be used. Preferred is a mixture of cross-linked polymer of acrylic acid and crystalline long chain acyl derivative.

Suspending agent will generally be present in a shampoo composition of the invention at levels of from 0.1 to 10%, preferably from 0.5 to 6%, more preferably from 0.9 to 4% by total weight of suspending agent based on the total weight of the composition.

### Conditioner Compositions

Another preferred product form for compositions in accordance with the invention is a conditioner for the treatment of hair (typically after shampooing) and subsequent rinsing.

Such conditioner compositions will typically comprise one or more conditioning surfactants which are cosmetically acceptable and suitable for topical application to the hair.

Suitable conditioning surfactants include those selected from cationic surfactants, used singly or in admixture. Preferably, the cationic surfactants have the formula N⁺R¹R²R³R⁴ wherein R¹, R², R³ and R⁴ are independently (C₁ to C₃₀) alkyl or benzyl. Preferably, one, two or three of R¹, R², R³ and R⁴ are independently (C₄ to C₃₀) alkyl and the other R¹, R², R³ and R⁹ group or groups are (C₁-C₆) alkyl or benzyl. More preferably, one or two of R¹, R², R³ and R⁴ are independently (C₆ to C₃₀) alkyl and the other R¹, R², R³ and R⁴ groups are (C₁-C₆) alkyl or benzyl groups. Optionally, the alkyl groups may comprise one or more ester (-OCO- or -COO-) and/or ether (-O-) linkages within the alkyl chain. Alkyl groups may optionally be substituted with one or more hydroxyl groups. Alkyl groups may be straight chain or branched and, for alkyl groups having 3 or more carbon atoms, cyclic. The alkyl groups may be saturated or may contain one or more carbon-carbon double bonds (eg, oleyl). Alkyl groups are optionally ethoxylated on the alkyl chain with one or more ethyleneoxy groups.

Suitable cationic surfactants for use in conditioner compositions as claimed in the invention include cetyltrimethylammonium chloride, behenyltrimethylammonium chloride, cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, dihydrogenated tallow dimethyl ammonium chloride (eg, Arquad 2HT/75 from Akzo Nobel), cocotrimethylammonium chloride, PEG-2-oleammonium chloride and the corresponding hydroxides thereof. Further suitable cationic surfactants include those materials having the CTFA designations Quaternium-5, Quaternium-31 and Quaternium-18. Mixtures of any of the foregoing materials may also be suitable. A particularly useful cationic surfactant for use in conditioners as claimed in the invention is cetyltrimethylammonium chloride, available commercially, for example as GENAMIN CTAC, ex Hoechst Celanese. Another particularly useful cationic surfactant for use in conditioners as claimed in the invention is behenyltrimethylammonium chloride, available commercially, for example as GENAMIN KDMP, ex Clariant.

Another example of a class of suitable cationic surfactants for use in the invention, either alone or together with one or more other cationic surfactants, is a combination of (i) and (ii) below:
(i) an amidoamine corresponding to the general formula (I): 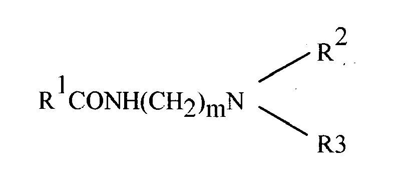 in which R¹ is a hydrocarbyl chain having 10 or more carbon atoms,
   R² and R³ are independently selected from hydrocarbyl chains of from 1 to 10 carbon atoms, and
   m is an integer from 1 to about 10; and
(ii) an acid.

As used herein, the term hydrocarbyl chain means an alkyl or alkenyl chain.

Preferred amidoamine compounds are those corresponding to formula (I) in which
R¹ is a hydrocarbyl residue having from about 11 to about 24 carbon atoms,
R² and R³ are each independently hydrocarbyl residues, preferably alkyl groups, having from 1 to about 4 carbon atoms, and
m is an integer from 1 to about 4.

Preferably, R² and R³ are methyl or ethyl groups.

Preferably, m is 2 or 3, i.e. an ethylene or propylene group.

Preferred amidoamines useful herein include stearamidopropyldimethylamine, stearamidopropyldiethylamine, stearamidoethyldiethylamine, stearamidoethyldimethylamine, palmitamidopropyldimethylamine, palmitamidopropyldiethylamine, palmitamidoethyldiethylamine, palmitamidoethyldimethylamine, behenamidopropyldimethylamine, behenamidopropyldiethylmine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, and mixtures thereof.

Particularly preferred amidoamines useful herein are stearamidopropyldimethylamine, stearamidoethyldiethylamine, and mixtures thereof.

Commercially available amidoamines useful herein include: stearamidopropyldimethylamine with tradenames LEXAMINE S-13 available from Inolex (Philadelphia Pennsylvania, USA) and AMIDOAMINE MSP available from Nikko (Tokyo, Japan), stearamidoethyldiethylamine with a tradename AMIDOAMINE S available from Nikko, behenamidopropyldimethylamine with a tradename INCROMINE BB available from Croda (North Humberside, England), and various amidoamines with tradenames SCHERCODINE series available from Scher (Clifton New Jersey, USA).

Acid (ii) may be any organic or mineral acid which is capable of protonating the amidoamine in the hair treatment composition. Suitable acids useful herein include hydrochloric acid, acetic acid, tartaric acid, fumaric acid, lactic acid, malic acid, succinic acid, and mixtures thereof. Preferably, the acid is selected from the group consisting of acetic acid, tartaric acid, hydrochloric acid, fumaric acid, and mixtures thereof.

The primary role of the acid is to protonate the amidoamine in the hair treatment composition thus forming a tertiary amine salt (TAS) in situ in the hair treatment composition. The TAS in effect is a non-permanent quaternary ammonium or pseudo-quaternary ammonium cationic surfactant.

Suitably, the acid is included in a sufficient amount to protonate all the amidoamine present, i.e. at a level which is at least equimolar to the amount of amidoamine present in the composition.

In conditioners of the invention, the level of cationic surfactant will generally range from 0.01 to 10%, more preferably 0.05 to 7.5%, most preferably 0.1 to 5% by weight of the composition.

Conditioners of the invention will typically also incorporate a fatty alcohol. The combined use of fatty alcohols and cationic surfactants in conditioning compositions is believed to be especially advantageous, because this leads to the formation of a lamellar phase, in which the cationic surfactant is dispersed.

Representative fatty alcohols comprise from 8 to 22 carbon atoms, more preferably 16 to 22. Fatty alcohols are typically compounds containing straight chain alkyl groups. Examples of suitable fatty alcohols include cetyl alcohol, stearyl alcohol and mixtures thereof. The use of these materials is also advantageous in that they contribute to the overall conditioning properties of compositions of the invention.

The level of fatty alcohol in conditioners of the invention will generally range from 0.01 to 10%, preferably from 0.1 to 8%, more preferably from 0.2 to 7%, most preferably from 0.3 to 6% by weight of the composition. The weight ratio of cationic surfactant to fatty alcohol is suitably from 1:1 to 1:10, preferably from 1:1.5 to 1:8, optimally from 1:2 to 1:5. If the weight ratio of cationic surfactant to fatty alcohol is too high, this can lead to eye irritancy from the composition. If it is too low, it can make the hair feel squeaky for some consumers.

### Further Conditioning Agents

Compositions of the invention may comprise further conditioning agents to optimise wet and dry conditioning benefits.

Particularly preferred further conditioning agents are silicone emulsions.

Suitable silicone emulsions include those formed from silicones such as polydiorganosiloxanes, in particular polydimethylsiloxanes which have the CTFA designation dimethicone, polydimethyl siloxanes having hydroxyl end groups which have the CTFA designation dimethiconol, and amino-functional polydimethyl siloxanes which have the CTFA designation amodimethicone.

The emulsion droplets may typically have a Sauter mean droplet diameter (D_{3,2}) in the composition of the invention ranging from 0.01 to 20 micrometer, more preferably from 0.2 to 10 micrometer.

A suitable method for measuring the Sauter mean droplet diameter (D_{3,2}) is by laser light scattering using an instrument such as a Malvern Mastersizer.

Suitable silicone emulsions for use in compositions of the invention are available from suppliers of silicones such as Dow Corning and GE Silicones. The use of such pre-formed silicone emulsions is preferred for ease of processing and control of silicone particle size. Such pre-formed silicone emulsions will typically additionally comprise a suitable emulsifier such as an anionic or nonionic emulsifier, or mixture thereof, and may be prepared by a chemical emulsification process such as emulsion polymerisation, or by mechanical emulsification using a high shear mixer. Pre-formed silicone emulsions having a Sauter mean droplet diameter (D_{3,2}) of less than 0.15 micrometers are generally termed microemulsions.

Examples of suitable pre-formed silicone emulsions include emulsions DC2-1766, DC2-1784, DC-1785, DC-1786, DC-1788 and microemulsions DC2-1865 and DC2-1870, all available from Dow Corning. These are all emulsions/microemulsions of dimethiconol. Also suitable are amodimethicone emulsions such as DC939 (from Dow Corning) and SME253 (from GE Silicones).

Also suitable are silicone emulsions in which certain types of surface active block copolymers of a high molecular weight have been blended with the silicone emulsion droplets, as described for example in WO03/094874. In such materials, the silicone emulsion droplets are preferably formed from polydiorganosiloxanes such as those described above. One preferred form of the surface active block copolymer is as described in the following formula: 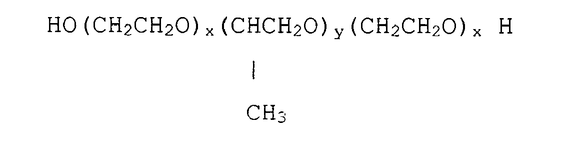 wherein the mean value of x is 4 or more and the mean value of y is 25 or more.

Another preferred form of the surface active block copolymer is as claimed in the following formula: 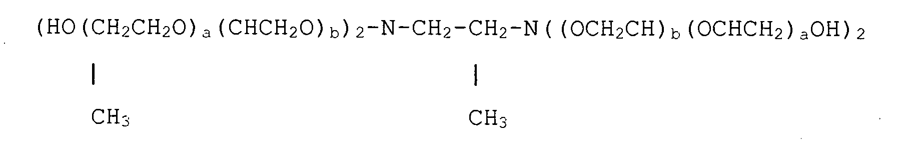 wherein the mean value of a is 2 or more and the mean value of b is 6 or more.

Mixtures of any of the above described silicone emulsions may also be used.

Silicone will generally be present in a composition of the invention at levels of from 0.05 to 10%, preferably 0.05 to 5%, more preferably from 0.5 to 2% by total weight of silicone based on the total weight of the composition.

### Other Optional Ingredients

A composition of the invention may contain other ingredients for enhancing performance and/or consumer acceptability. Such ingredients include fragrance, dyes and pigments, pH adjusting agents, pearlescers or opacifiers, viscosity modifiers, preservatives, and natural hair nutrients such as botanicals, fruit extracts, sugar derivatives and amino acids.

The invention will now be described with reference to the following non-limiting examples. In the examples and throughout this specification, all percentages are by weight based on total composition and based on active material unless indicated otherwise.

### EXAMPLES

Example 1. The one-pot synthesis and loading of a core-shell brush copolymer from small molecule reactants, by tandem ROMP and RAFT copolymerisation.

The one-pot synthesis and loading of a nanocage from small molecule reactants, by tandem ROMP and RAFT copolymerisation is shown in Scheme 2 below.

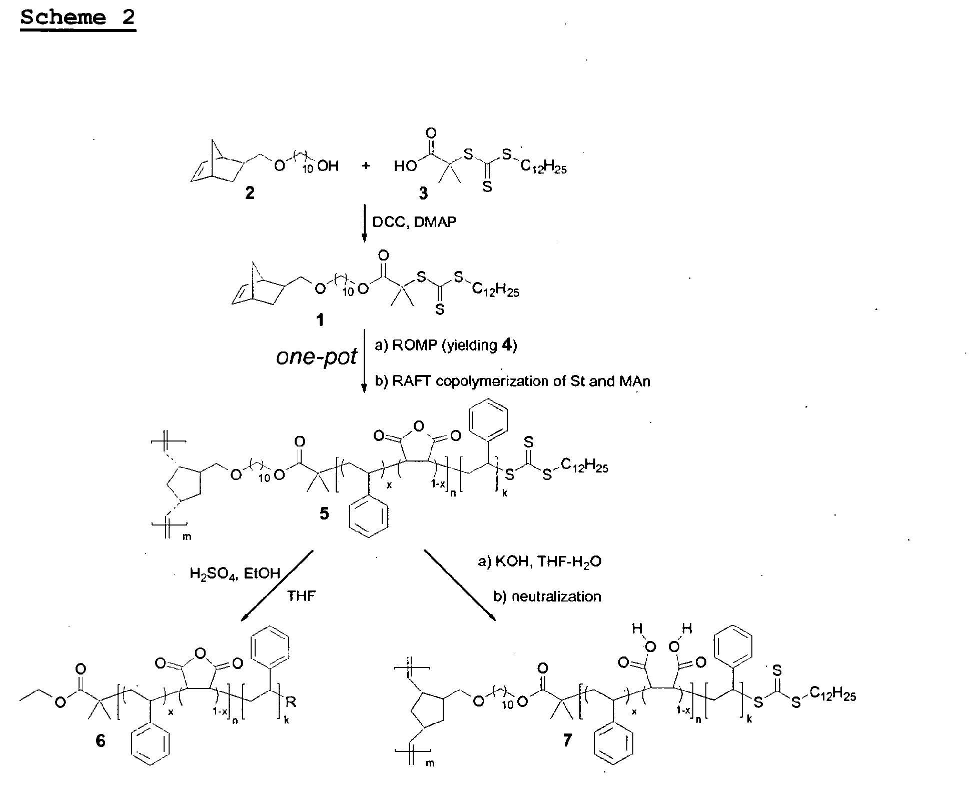

As a critical agent for the tandem synthesis of core-shell brush copolymer, an exo-norbornene-functionalized RAFT agent **1** was prepared in 87% yield by esterification of a norbornene-functionalized alcohol **2** with an acid-functionalized RAFT agent **3** (2.0 eq), using *N,N'-*dicyclohexylcarbodimide (DCC; 2.1 eq), and 4-(dimethylamino)pyridine (DMAP; 0.2 eq) in CH₂Cl₂ at room temperature for 11 h.^{20 1}H NMR analysis of **1** showed a series of characteristic resonances, including those of norbornene alkene protons *a* and *b* (at 6.03-6.16 ppm), C*H*₂OCO protons *e* (at 4.07 ppm), C*H*₂O*CH*₂ protons *c* and *d* and SC*H*₂ protons *f* (at 3.20-3.55 ppm), and C*H*₃ protons *g* (at 0.87 ppm). Their integration area ratios of 1.95:2.00:5.95:3.10 were in excellent agreement with the number ratio of protons of 2:2:6:3, verifying the molecular structure of **1.** One-pot preparation of core-shell brush copolymer from small molecule reactants was then performed by ROMP of the *exo-*norbornene-functionalized RAFT agent **1**, followed by using the resulting polyfunctional RAFT agent and AIBN (as initiator) for "grafting from" *via* RAFT copolymerization of styrene (St) and maleic anhydride (MAn). The ROMP of **1** (99.7 mg) was conducted, using Grubbs' catalyst RuCl₂(CHC₆H₅) [P(C₆H₁₁)₃]₂ (0.02 eq) in CH₂Cl₂ at room temperature for 1 h. ¹H NMR and GPC analyses of an aliquot of the reaction mixture (19 vol%, terminated by ethyl vinyl ether) determined the successful transformation of **1** into a well-defined polyfunctional RAFT agent. Near complete conversion (>99%) of **1** was verified by essential absence of ¹H NMR resonances of norbornene alkene protons of **1** at 6.03-6.16 ppm, and the formation of poly (**1**), *i.e.* **4,** .was supported by a series of characteristic resonances, including those of alkene protons *a*' and *b*' of the poly(norbornene)-based main-chain (at 5.00-5.50 ppm), C*H*₂OCO protons *e'* (at 4.07 ppm), C*H*₂OC*H*₂ protons *c'* and *d'* and SC*H*₂ protons *f'* (at 3.20-3.55 ppm), and C*H*₃ protons *g'* (at 0.87 ppm). Their integration area ratios of 1.93:2.00:5.94:3.03 agreed very well with the number ratios of protons of 2:2:6:3, indicating quantitatively one RAFT functionality per repeal unit of **4.** By GPC, **4** was found to have a *Mₙ* of 40.6 kDa (40600 atomic mass units (U)), and a low polydisperisty index of 1.24. Relative to a calculated *Mₙ* value of 31.5 kDa, (31500 atomic mass units (U)), the experimental *Mₙ* value of **4** indicated an initiation efficiency of 78 %.

By hydrolyzing the MAn units in the poly(St-*stat*-MAn) blocks into hydrophilic maleic acid units, **5** could be further converted into amphiphilic core-shell brush copolymer **7.** Hydrolysis proceeded readily at room temperature under basic conditions using KOH (potassium hydroxide) to promote the reaction. Subsequent neutralization of the reaction solution gave **7.** Both ¹H NMR and FT-IR spectroscopic characterizations were used to compare **7** with its precursor **5.** ¹H NMR resonances of carboxylic protons centered at 12.0 ppm were observed by ¹H NMR measurement **of 7** in DMSO-*d₆*, verifying the presence of maleic acid units in **7.** Critical differences between **7** and **5** were revealed by FT-IR. Core-shell brush copolymer **5** showed two C=O stretching frequencies at 1857 and 1778 cm⁻¹ for its cyclic anhydride groups and an absence of an O-H stretching absorbance. However, amphiphilic core-shell brush copolymer **7** possessed only one C=O stretching frequency at 1714 cm⁻¹ and a broad O-H stretching absorption at 2500-3500 cm⁻¹, indicating complete functional group transformation from the anhydrides to carboxylic acid groups. Additionally, different solubilities for **7** relative to **5** were found. For example, **5** was soluble in CDCl₃, but **7** was insoluble in CDCl₃ and could be dissolved by 1:2 CDCl₃-CD₃OD.

A nanocage prepared by crosslinking the amphiphilic core-shell brush copolymer brush 7 and loaded with 15 wt% salicylic acid, was synthesised following the above Scheme This sample, nanocage loaded with 15 wt% salicylic acid, was synthesized following Scheme 1. The amphiphilic core-shell brush copolymer **7** has a polynorbornene-based backbone, polyisoprene core, and poly(acrylic acid) shell. The shell cross-linking reaction of **7** was carried out using 0.11 eq of 2,2'-(ethylenedioxy)bis(ethylamine) cross-linker and 0.22 eq of 1-[3'-(dimethylamino)propyl]-3-ethylcarbodiimide methiodide (a catalyst) relative to the acrylic acid shell units of **7.** The polyisoprene core of the resulting shell cross-linked nanoparticle **8** was then degraded by ozone treatment, followed by reduction with Na₂SO₃. The nanocage **9** formed was then loaded with salicylic acid (~15 wt%) by stirring the 20% *p*-dioxane-water solution over 2 days, and then the solution was lyophilized to give dry sample.

The loading of salicylic acid into nanocage was proven by ¹H NMR analysis (spectra attached). The nanocage sample loaded with salicylic acid also showed IR absorption at 3233 cm⁻¹, which was absent for nanocage **9.** Tapping-mode AFM measurements indicated that there is detectable but not significant size change for nanocages before and after loading salicylic acid. Before loading, the nanocages have diameters ranging from 20 to 45 nm, with heights below 1.5 nm on mica; after loading, the nanocages have diameters ranging from 20 to 50 nm, with heights below 1.5 nm on mica. As a note, the water-solubility of nanocage sample can decrease due to lyophilization.

The following are examples of compositions as claimed in the invention.

The materials used in the examples include the following:

| **Material** | **Supplier** | **Function** |
|---|---|---|
| Silicone emulsion X2 1787^{™} | Dow Corning | Conditioning |
| VOLPO CS 50^{™} | Croda | Chemicals |
| surfactant | | |
| Sepicide LD^{™} | Seppic | Preservative |
| Cremophor RH410^{™} | BASF | Stabiliser |
| Silicone DC 200/DC 24 S^{™} | Dow Corning | Conditioning |
| Silwet L7602/L-720^{™} | Union Carbide | Surfactant |
| CAP 40^{™} | Calor Gas | Propellant |
| Carbopol 980^{™} | BF Goodrich | Structurant |
| Jaguar HP-105^{™} | Rhodia | Conditioning |
| Silicone Fluid 245^{™} | Dow Corning | Conditioning |
| Luviset PUR | BASF | Adhesive |
| DynamX^{®} (28-019A) | National Starch | Adhesive |
| Polyurethane-14 (and) AMP-Acrylates Copolymer | | |
| Ethanol used as SD Alcohol 40-B (92% active) | | |

### Example 2

### A Shampoo formulation

| **Formulation ingredients** | **Weight%** |
|---|---|
| Sodium laurylether sulphate (2EO) | 12 |
| Cocoyl amidopropyldimethyl glycine | 2 |
| Silicone emulsion | 2 |
| Nanaocage loaded with salicylic acid* | 2.80 |
| Guar hydroxypropyl trimethylammonium | 0.30 |
| chloride | |

| | |
|---|---|
| * according to Example 1 | |

### Example 3

A styling mousse is formulated as follows

| **Material** | % in product (w/w) |
|---|---|
| Silicone Emulsion X2 1787 | 1.2 |
| DynamX^{®} (28-019A) Polyurethane-14 (and) AMP-Acrylates Copolymer | 1.5 |
| VOLPO CS 50 | 0.3 |
| Nanaocage loaded with salicylic acid* | 1.85 |
| Sepicide LD | 0.4 |
| Cremophor RH410 | 0.2 |
| Ethanol | 7.5 |
| CAP 40 | 8.0 |
| Perfume | 0.2 |
| Water | to 100% |

| | |
|---|---|
| * according to Example 1 | |

## Claims

1. A composition comprising a brush copolymer of formula 1: 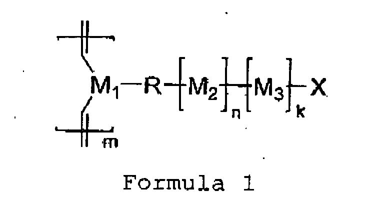 in which M₁ is a unit obtainable from ring opening metathesis polymerisation (ROMP);
R is an alkyl, ether, ester or aryl unit;
M₂ and M₃ are independently selected from units obtainable by reversible addition fragmentation chain transfer polymerization(RAFT);
X is a terminal unit selected from the group consisting of dithioester, trithiocarbonate, xanthate; and
m is an integer from 2 to 1 million, n is an integer from 2 to 500,000 and k is an integer from 2 to 500,000; in which incorporated within the core shell brush copolymer is a benefit agent selected from hair styling polymers, colourants, hair conditioners, hair cleansers, hair growth promoters, permanent wave compounds, hair relaxers, amino acids, vitamins, hair straighteners, hair growth stimulants, antibacterial compounds, antifungal compounds, anti-inflammatory compounds, sunscreens and mixtures thereof.

2. A composition as claimed in claim 1 in which M1 of the brush copolymer is a unit obtainable from the ring opening metathesis polymerisation (ROMP) of cyclooctadiene or norbornene.

3. A composition as claimed in claim 1 or claim 2 in which M₂ and/or M₃ of the brush copolymer are independently selected from units obtainable by reversible addition fragmentation chain transfer polymerization (RAFT) of styrene, isoprene, methyl acrylate, *tert*-butyl acrylate, dimethyl acryamide, acrylic acid, acrylonitrile, methyl methacrylate, maleic anhydride, vinyl acetate, vinyl pyridine or vinyl phenyl ketone.

4. A composition as claimed in any preceding claim in which the core-shell brush copolymer is of formula 2: 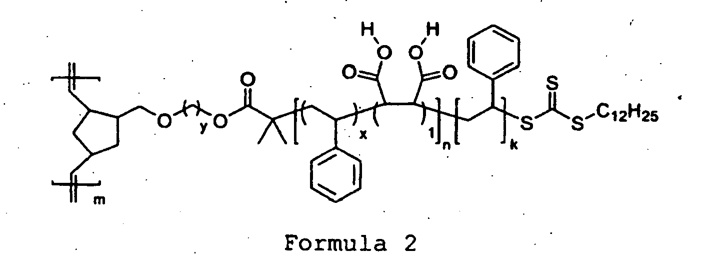 in which m is an integer from 2 to 1 million, y is an integer form 2 to 18, × is an integer from 1 to 100, n is an integer from 2 to 500,000 and k is an integer from 2 to 500,000.

5. Composition as claimed in any preceding claim, in which the composition comprises the core shell brush copolymer in an amount of from 0.01% to 50%.

6. Composition as claimed in claim 1 in which the benefit agent is selected from the group consisting of an antifungal compound, antibacterial compound, an anti-inflammatory compound or mixtures thereof.

7. Composition as claimed in claim 6 in which the benefit agent is salicylic acid.

8. Composition as claimed in any preceding claim which is a rinse off product.

9. Composition as claimed in Claim 11 which is a shampoo or conditioner.

10. Use of a composition comprising a core-shell brush copolymer having the structure of formula 1: 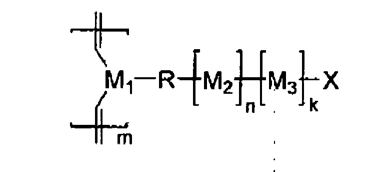 in which M₁ is a unit obtainable from ring opening metathesis polymerisation (ROMP);
R is an alkyl, ether, ester or aryl unit;
M₂ and M₃ are independently selected from units obtainable by reversible addition fragmentation chain transfer polymerization(RAFT);
X is a terminal unit selected from the group consisting of dithioester, trithiocarbonate, xanthate; and
m is an integer from 2 to 1 million, n is an integer from 2 to 500,000 and k is an integer from 2 to 500,000;
to deliver a benefit agent to the hair.

11. A method of treating the hair by application to the hair a composition comprising a core-shell brush copolymer having the structure of formula 1: 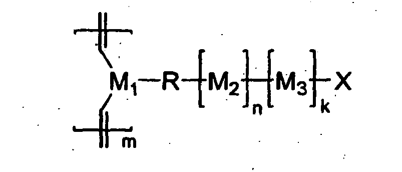 in which M₁ is a unit obtainable from ring opening metathesis polymerisation (ROMP);
R is an alkyl, ether, ester or aryl unit;
M₂ and M₃ are independently selected from units obtainable by reversible addition fragmentation chain transfer polymerization (RAFT);
X is a terminal unit selected from the group consisting of dithioester, trithiocarbonate, xanthate; and
m is an integer from 2 to 1 million, n is an integer from 2 to 500,000 and k is an integer from 2 to 500,000.

## Patentansprüche

1. Zusammensetzung, umfassend ein Bürsten-Copolymer der Formel 1: 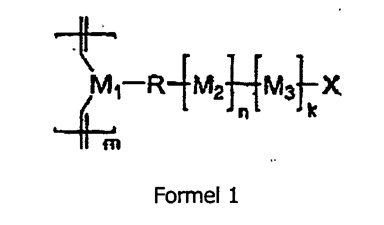 in welcher M₁ eine Einheit ist, die durch Ringöffnungs-Metathese-Polymerisation (ROMP) erhältlich ist;
R eine Alkyl-, Ether-, Ester- oder Aryl-Einheit ist;
M₂ und M₃ unabhängig ausgewählt sind aus Einheiten, die durch reversible Additions-Fragmentierungs-Kettentransfer-Polymerisation (RAFT) erhältlich sind;
X eine terminale Einheit ist, ausgewählt aus der Gruppe, bestehend aus Dithioester, Trithiocarbonat, Xanthat; und
m eine ganze Zahl von 2 bis 1 Million ist, n eine ganze Zahl von 2 bis 50 000 ist und k eine ganze Zahl von 2 bis 500 000 ist; in die innerhalb des Kern-Hülle-Bürsten-Copolymers ein Wirkstoff ist, der ausgewählt ist aus Haarstyling-Polymeren, Haarfarben, Haarkonditionern, Haarreinigungsmitteln, Haarwachstumsförderern, Dauerwellenverbindungen, Haarglättungsmitteln, Aminosäuren, Vitaminen, Haarglättungsmitteln (hair straighteners), Stimulantien für das Haarwachstum, anti-bakteriellen Verbindungen, anti-fungalen Verbindungen, anti-inflammatorischen Verbindungen, Lichtschutzmitteln und Gemischen davon.

2. Zusammensetzung, wie sie in Anspruch 1 beansprucht wird, wobei M1 des Bürsten-Copolymers eine Einheit ist, die aus Ringöffnungs-Metathese-Polymerisation (ROMP) von Cyclooctadien oder Norbornen erhältlich ist.

3. Zusammensetzung, wie sie in Anspruch 1 oder Anspruch 2 beansprucht wird, wobei M₂ und/oder M₃ des Bürsten-Copolymers unabhängig ausgewählt sind aus Einheiten, die durch reversible Additions-Fragmentierungs-Kettentransfer-Polymerisation (RAFT) von Styrol, Isopren, Methylacrylat, tert-Butylacrylat, Dimethylacrylamid, Acrylsäure, Acrylonitril, Methylmethacrylat, Maleinsäureanhydrid, Vinylacetat, Vinylpyridin oder Vinylphenylketon erhältlich sind.

4. Zusammensetzung, wie sie in einem vorangehenden Anspruch beansprucht wird, wobei das Kern-Hülle-Bürsten-Copolymer die Formel 2 hat: 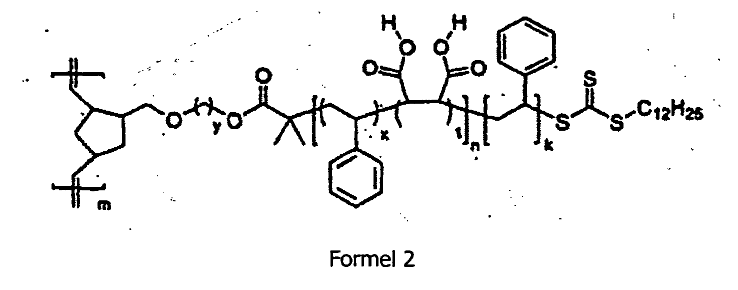 worin m eine ganze Zahl von 2 bis 1 Million ist, y eine ganze Zahl von 2 bis 18 ist, x eine ganze Zahl von 1 bis 100 ist, n eine ganze Zahl von 2 bis 500 000 ist und k eine ganze Zahl von 2 bis 500 000 ist.

5. Zusammensetzung, wie sie in einem vorangehenden Anspruch beansprucht wird, wobei die Zusammensetzung das Kern-Hülle-Bürsten-Copolymer in einer Menge von 0,01 % bis 50 % umfasst.

6. Zusammensetzung, wie sie in Anspruch 1 beansprucht wird, wobei der Wirkstoff ausgewählt ist aus der Gruppe, bestehend aus einer anti-fungalen Verbindung, einer anti-bakteriellen Verbindung, einer anti-inflammatorischen Verbindung und Gemischen davon.

7. Zusammensetzung, wie sie in Anspruch 6 beansprucht wird, wobei der Wirkstoff Salicylsäure ist.

8. Zusammensetzung, wie sie in einem vorangehenden Anspruch beansprucht wird, welche ein Produkt zum Ausspülen ist.

9. Zusammensetzung, wie sie in Anspruch 11 beansprucht wird, welche ein Shampoo oder ein Konditioner ist.

10. Verwendung einer Zusammensetzung, umfassend ein Kern-Hülle-Bürsten-Copolymer, das die Struktur der Formel 1 hat: 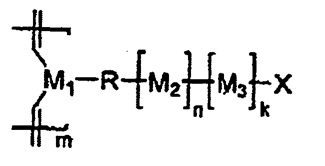 in welcher M₁ eine Einheit ist, die durch Ringöffnungs-Metathese-Polymerisation (ROMP) erhältlich ist;
R eine Alkyl-, Ether-, Ester- oder Aryl-Einheit ist;
M₂ und M₃ unabhängig ausgewählte Einheiten sind, die durch reversible Additions-Fragmentierungs-Kettentransfer-Polymerisation (RAFT) erhältlich sind;
X eine terminale Einheit ist, ausgewählt aus der Gruppe, bestehend aus Dithioester, Trithiocarbonat, Xanthat; und
m eine ganze Zahl von 2 bis 1 Million ist, n eine ganze Zahl von 2 bis 500 000 ist und k eine ganze Zahl von 2 bis 500 000 ist;
zur Abgabe eines Wirkstoffs an das Haar.

11. Verfahren zur Behandlung des Haars durch Auftragen bzw. Anwenden auf das Haar einer Zusammensetzung, umfassend ein Kern-Hülle-Bürsten-Copolymer mit der Struktur der Formel 1: 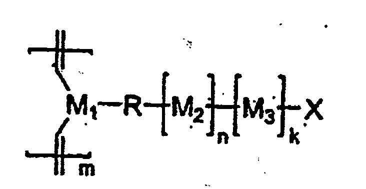 worin M₁ eine Einheit ist, die durch Ringöffnungs-Metathese-Polymerisation (ROMP) erhältlich ist;
R eine Alkyl-, Ether-, Ester- oder Aryl-Einheit ist;
M₂ und M₃ unabhängig ausgewählt sind aus Einheiten, die durch reversible Additionsfragmentierungs-Kettentransfer-Polymersation (RAFT) erhältlich sind;
X eine terminale Einheit ist, ausgewählt aus der Gruppe, bestehend aus Dithioester, Trithiocarbonat, Xanthat; und
m eine ganze Zahl von 2 bis 1 Million ist, n eine ganze Zahl von 2 bis 500 000 ist und k eine ganze Zahl von 2 bis 500 000 ist.

## Revendications

1. Composition comprenant un copolymère en peigne de formule 1 : 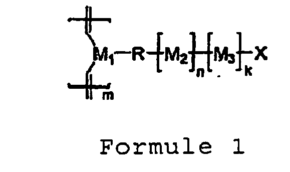 dans laquelle M₁ est un motif pouvant être obtenu par polymérisation par métathèse par ouverture de cycle (ROMP) ;
R est un motif alkyle, éther, ester ou aryle ;
M₂ et M₃ sont indépendamment choisis parmi les motifs pouvant être obtenus par polymérisation de transfert de chaîne avec addition-fragmentation réversible (TCAR) ;
X est un motif terminal choisi dans le groupe constitué par le dithioester, le trithio-carbonate, le xanthate ; et
m est un nombre entier de 2 à 1 000 000, n est un nombre entier de 2 à 500 000 et k est un nombre entier de 2 à 500 000 ; dans laquelle est incorporé dans le copolymère en peigne coeur-coque un agent bénéfique choisi parmi les polymères de coiffure, les colorants, les tonifiants capillaires, les nettoyants capillaires, les activateurs de croissance capillaire, les composés pour permanente, les produits défrisants, les acides aminés, les vitamines, les produits lissants, les stimulants de croissance capillaire, les composés antibactériens, les composés antifongiques, les composés anti-inflammatoires, les écrans solaires et leurs mélanges.

2. Composition selon la revendication 1, dans laquelle M₁ du copolymère en peigne est un motif pouvant être obtenu par polymérisation par métathèse par ouverture de cycle (ROMP) de cyclooctadiène ou norbornène.

3. Composition selon la revendication 1 ou la revendication 2, dans laquelle M₂ et/ou M₃ du polymère en peigne sont indépendamment choisis parmi les motifs pouvant être obtenus par polymérisation de transfert de chaîne avec addition-fragmentation réversible (TCAR) de styrène, isoprène, acrylate de méthyle, acrylate de tert-butyle, diméthylacrylamide, acide acrylique, acrylonitrile, méthacrylate de méthyle, anhydride maléique, acétate de vinyle, vinylpyridine ou vinylphénylcétone.

4. Composition selon l'une quelconque des revendications précédentes, dans laquelle le copolymère en peigne coeur-coque est représenté par la formule 2 : 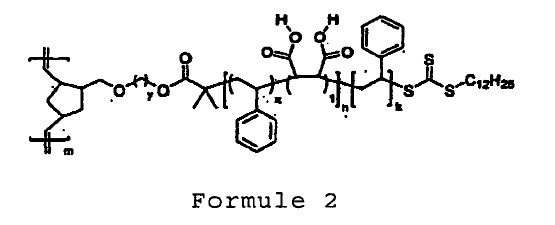 dans laquelle m est un nombre entier de 2 à 1 000 000, y est un nombre entier de 2 à 18, x est un nombre entier de 1 à 100, n est un nombre entier de 2 à 500 000 et k est un nombre entier de 2 à 500 000.

5. Composition selon l'une quelconque des revendications précédentes, dans laquelle la composition comprend le copolymère en peigne coeur-coque en une quantité de 0,01 % à 50 %.

6. Composition selon la revendication 1, dans laquelle l'agent bénéfique est choisi dans le groupe constitué par un composé antifongique, un composé antibactérien, un composé anti-inflammatoire ou leurs mélanges.

7. Composition selon la revendication 6, dans lequel l'agent bénéfique est l'acide salicylique.

8. Composition selon l'une quelconque des revendications précédentes, qui est un produit rinçable.

9. Composition selon la revendication 11, qui est un shampooing ou un après-shampooing.

10. Utilisation d'une composition comprenant un copolymère en peigne coeur-coque ayant la structure de formule 1 : 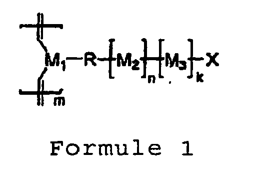 dans laquelle M₁ est un motif pouvant être obtenu par polymérisation par métathèse par ouverture de cycle (ROMP) ;
R est un motif alkyle, éther, ester ou aryle ;
M₂ et M₃ sont indépendamment choisis parmi les motifs pouvant être obtenus par polymérisation de transfert de chaîne avec addition-fragmentation réversible (TCAR) ;
X est un motif terminal choisi dans le groupe constitué par le dithioester, le trithio-carbonate, le xanthate ; et
m est un nombre entier de 2 à 1 000 000, n est un nombre entier de 2 à 500 000 et k est un nombre entier de 2 à 500 000 ;
pour délivrer un agent bénéfique aux cheveux.

11. Procédé de traitement des cheveux par l'application sur les cheveux d'une composition comprenant un copolymère en peigne coeur-coque ayant la structure de formule 1 : 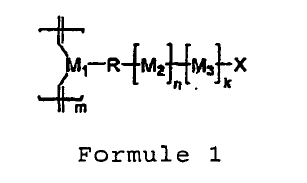 dans laquelle M₁ est un motif pouvant être obtenu par polymérisation par métathèse par ouverture de cycle (ROMP) ;
R est un motif alkyle, éther, ester ou aryle ;
M₂ et M₃ sont indépendamment choisis parmi les motifs pouvant être obtenus par polymérisation de transfert de chaîne avec addition-fragmentation réversible (TCAR) ;
X est un motif terminal choisi dans le groupe constitué par le dithioester, le trithio-carbonate, le xanthate ; et
m est un nombre entier de 2 à 1 000 000, n est un nombre entier de 2 à 500 000 et k est un nombre entier de 2 à 500 000.
